# EUROPEAN PATENT SPECIFICATION

(11) **EP 1 644 008 B1**
(45) Date of publication and mention of the grant of the patent: **21.12.2011**
(21) Application number: 04753737.8
(22) Date of filing: 01.06.2004
(51) Int. Cl.: A61K 31/635

(54) **CONTINUOUS DOSING REGIMEN WITH ABT-751**
KONTINUIERLICHES DOSIERSCHEMA MIT ABT-751
SCHEMA POSOLOGIQUE D'ADMINISTRATION EN CONTINU

(30) Priority: 29.05.2003 US 447588; 10.05.2004 US 842667; 28.05.2004 US 857235
(43) Date of publication of application: 12.04.2006
(73) Proprietor: ABBOTT LABORATORIES, Abbott Park, IL 60064-6008 (US); Meek, Kysa, A., McHenry, IL 60050 (US)
(72) Inventor: GORDON, Gary, Highland Park, IL 60035 (US); HAGEY, Anne, E., Lake Forest, IL 60045 (US); ROSENBERG, Saul, H., Grayslake, IL 60030 (US)
(74) Representative: Modiano, Micaela Nadia
(86) International application number: PCT/US2004/016973
(87) International publication number: WO 2004/105794

(56) References cited:
- DATABASE MEDLINE [Online] US NATIONAL LIBRARY OF MEDICINE (NLM), BETHESDA, MD, US; 1998, YAMAMOTO K ET AL: "Phase I study of E7010." XP002308547 Database accession no. NLM9654112 & CANCER CHEMOTHERAPY AND PHARMACOLOGY. 1998, vol. 42, no. 2, 1998, pages 127-134, ISSN: 0344-5704
- "A phase I trial of ABT-751, a novel microtubulin inhibitor" EUROPEAN JOURNAL OF CANCER, PERGAMON PRESS, OXFORD, GB, vol. 38, November 2002 (2002-11), page S42, XP004403567 ISSN: 0959-8049
- DATABASE MEDLINE [Online] US NATIONAL LIBRARY OF MEDICINE (NLM), BETHESDA, MD, US; 1 April 1994 (1994-04-01), KOYANAGI N ET AL: "In vivo tumor growth inhibition produced by a novel sulfonamide, E7010, against rodent and human tumors." XP002308548 Database accession no. NLM8137285 & CANCER RESEARCH. 1 APR 1994, vol. 54, no. 7, 1 April 1994 (1994-04-01), pages 1702-1706, ISSN: 0008-5472
- DATABASE MEDLINE [Online] US NATIONAL LIBRARY OF MEDICINE (NLM), BETHESDA, MD, US; 2001, FUNAHASHI Y ET AL: "Effect of E7010 on liver metastasis and life span of syngeneic C57BL/6 mice bearing orthotopically transplanted murine Colon 38 tumor." XP002308549 Database accession no. NLM11269745 & CANCER CHEMOTHERAPY AND PHARMACOLOGY. 2001, vol. 47, no. 2, 2001, pages 179-184, ISSN: 0344-5704
- DATABASE MEDLINE [Online] US NATIONAL LIBRARY OF MEDICINE (NLM), BETHESDA, MD, US; October 2000 (2000-10), HAYAKAWA K ET AL: "Collaborative work to evaluate toxicity on male reproductive organs by repeated dose studies in rats 17). Testicular toxicity of E7010, a sulfonamide tubulin polymerization inhibitor." XP002308550 Database accession no. NLM11349441 & THE JOURNAL OF TOXICOLOGICAL SCIENCES. OCT 2000, vol. 25 Spec No, October 2000 (2000-10), pages 173-178, ISSN: 0388-1350

## Description

### FIELD OF THE INVENTION

This invention pertains to a composition comprising cancer drugs, continuous oral dosing schedule with a drug which binds to the colchicine site of tubulin β-subunits, and compositions for use in treating cancer using continuous dosing schedules.

### BACKGROUND OF THE INVENTION

The efficacy of many commercially-available, parenterally administered tubulin β-subunit binders is compromised by the necessity of intermittent administration due to severity of coincident adverse side effects. There is therefore an existing need in the therapeutic arts for improved treatment of diseases with drugs which bind to tubulin β-subunits.

Yamamoto K et al, "Phase I study of E7010", Cancer Chemother. Pharmacol (1998), vol. 42, pages 127-134; "A phase I trial of ABT-751, a novel microtubulin inhibitor", European Journal of Cancer, vol. 38, November 2002, page S42; and Sprague E et al, "Phase I study of 21-day continuous dosing of the oral antimitotic agent ABT-751", Proceedings of American Society of Clinical Oncology, vol. 22, 2003, page 129, abstract no. 518 disclose the oral anti-tumor effectiveness of ABT-751 (N-(2-((4-hydroxyphenyl)amino)pyrid-3-yl)-4-methoxybenzenesulfonamide) in clinical trials with human cancer patients.

### SUMMARY OF THE INVENTION

The present invention provides a pharmaceutical composition having therapeutic synergy comprising N-(2-((4-hydroxyphenyl)amino)pyrid-3-yl)-4-methoxybenzenesulfonamide and at least one cancer drug selected from the group consisting of cisplatin, docetaxel, and 5-fluorouracil. In particular, the present invention provides such composition for use in the treatment of cancer.

In addition, the present invention provides the use of N-(2-((4-hydroxyphenyl)amino)pyrid-3-yl)-4-methoxybenzenesulfonamide and at least one additional drug selected from the group consisting of cisplatin, docetaxel, and 5-fluorouracil for the manufacture of a medicament for treating cancer in a human wherein said medicament is to be administered in a therapeutically acceptable amount.

### DETAILED DESCRIPTION OF THE INVENTION

The term "at least five days," as used herein, means the time period over which the drug is administered. In a preferred embodiment for the practice of this invention, at least five days means for the first 7 days of a 21 day schedule, for the first 14 days of a 21 day schedule, for he first 15 days of a 21 day schedule, for the first 21 days of a 28 day schedule, for 5 days then cessation for 5 days then continuation for 5 days then cessation for 5 days, i.e. (5 days on/5 days off) ×2, and for 7 days then cessation for 7 days then continuation for 7 days then cessation for 7 days, i.e. (7 days on/7 days off) ×2.

The term "colchicine site binder," as used herein, means a tubulin β-subunit binder which binds to the colchicine site of the tubulin β-subunits and thereby inhibits the polymerization of tubulin.

The drug which binds to the colchicine site of tubulin β-subunits for the practice of this invention is N-(2-((4-hydroxyphenyl)amino)pyrid-3-yl)-4-methoxybenzenesulfonamide, also referred to herein as N-(2-((4-hydroxyphenyl)amino)pyrid-3-yl)-4-methoxybenzenesulfonamide. The synthesis of N-(2-((4-hydroxyphenyl)amino)pyrid-3-yl)-4-methoxybenzenesulfonamide is taught in United States Patent No. 5,292,758, column 23, line 61 to column 24, line 12.

The term "cancer," as used herein, means bone marrow dyscrasias, breast (ductal and lobular) cancer, cervical cancer, colon cancer, leukemia, lung (small cell and non-small cell) cancer, lymphoma, melonoma, mouth and tongue cancer, pancreatic cancer, prostate cancer, rectal cancer, renal cancer, sarcoma, stomach cancer, uterine cancer, and cancers resulting from the metastasis of disease from these areas.

The term "continuous," as used herein, means at least once per day without missing a day.

The term "disease," as used herein, means an adverse physiological event. For the practice of this invention, the disease to be treated is cancer.

The term "drug," as used herein, means a compound which is suitable for prevention or treatment of disease or inhibition of one or more adverse physiological events.

Examples of parenterally administered drugs include vinca alkaloids (vincristine, vinblastine, and vinorelbine), taxanes (paclitaxel and docetaxel), 5-fluorouracil, cisplatin, docetaxel, gemcitabine, and colchicine site binders such as colchicine itself which is used to treat gouty arthritis.

The term "essentially reduced," as used herein in reference to severity of an adverse side effect means at least about 50% of the patient population tested did not experience that side effect at the Grade III or IV level, preferably about 75% of the patient population tested did not experience that side effect at the Grade III or IV level, more preferably about 85% of the patient population tested did not experience that side effect at the Grade III or IV level, even more preferably, about 95% of the patient population tested did not experience that side effect at the Grade III or IV level, and most preferably, 100% of the patient population tested did not experience that side effect at the Grade III or IV level.

The term "therapeutic synergy," as used herein, means a combination of two or more drugs having a therapeutic effect greater than the additive effect of each respective drug.

Binding affinities of N-(2-((4-hydroxyphenyl)amino)pyrid-3-yl)-4-methoxybenzenesulfonamide, vinblastine, and paclitaxel were evaluated using the competition of [³H]colchicine to biotinylated bovine brain tubulin in a scintillation proximity assay.

**TABLE 1**

| Inhibition Constants of ABT-751 and Other Tubulin β-Subunit Binders in Binding Experiments with Bovine Brain Tubulin | |
|---|---|
| Compound | Kᵢ (µM) |
| ABT-751 | 2.60 (n=4) |
| colchicine | 0.78 (n=7) |
| paclitaxel | >100 (n=4) |
| vinblastine | >100 (n=4) |

The data in TABLE 1 demonstrate that N-(2-((4-hydroxyphenyl)amino)pyrid-3-yl)-4-methoxybenzenesulfonamide displaces [³H]colchicine from the colchicine site of tubulin β-subunits and is therefore a colchicine site binder.

The data in TABLE 1 also demonstrate that vinblastine and paclitaxel do not displace [³H] colchicine, are therefore not colchicine-site binders, and therefore must bind to tubulin β-subunits sites which are different from the colchicine binding site.

N-(2-((4-hydroxyphenyl)amino)pyrid-3-yl)-4-methoxybenzenesulfonamide is a colchicine site binder and exemplifies drugs which are useful for treatment of diseases which may be treated with colchicine-site binders other than colchicine itself.

The effectiveness of colchicine-site binders as drugs which are useful for treatment of diseases in humans depends on variables such as the composition comprising the drug, its route of administration, the amount of drug administered, and the dosing schedule. This disclosure pertains to an unexpected and surprising combination of variables which lead to a favorable therapeutic event with a sufficient reduction in the severity of at least one adverse side effect selected from the group consisting of anemia, alopecia, fluid retention, myelosupression, neuropathy and neutropenia as compared to the same side effect coincident with treatment of the substantially same disease with a parenterally administered drug which binds to tubulin β-subunits.

M5076 is a transplantable murine reticulum cell sarcoma. N-(2-((4-hydxoxyphenyl)amino)pyrid-3-yl)-4-methoxybenzenesulfonamide exhibited significant antitumor activity in this syngeneic flank tumor model when administered orally once a day for 5 days. At its approximate MTD of 150 mg/kg for 5 days, N-(2-((4-hydroxyphenyl)amino)pyrid-3-yl)-4-methoxybenzenesulfonamide significantly inhibited tumor growth with T/C (tumor mass of test group divided by tumor mass of control group) and ILS (percent increase in life span) values of 13 and 42%, respectively. In contrast, this model was resistant to paclitaxel. Vincristine and doxorubicin were only marginally active (ILS = 17% and 13% respectively), while this model proved sensitive to cyclophosphamide.

N-(2-((4-hydroxyphenyl)amino)pyrid-3-yl)-4-methoxybenzenesulfonamide was evaluated against C26 colon tumors grown in the flank of CDF-1 mice. While only marginally active when administered on a 5-day schedule, extended dosing produced a significant antitumor response that was equivalent to that achieved with BCNU at the MTD. Paclitaxel was not efficacious against this tumor.

*Apc^{Min}* (Min) mice are models for genetically inherited intestinal cancer. These mice carry a dominant germline mutation in the *Apc* tumor suppressor gene that predisposes them to the development of numerous (>50) tumors throughout the intestinal tract.

N-(2-((4-hydroxyphenyl)amino)pyrid-3-yl)-4-methoxybenzenesulfonamide given orally on a once-a-day schedule for 28 days at 150 mg/kg/day led to a significant reduction in tumor burden in Min mice. The average tumor number for treated mice was 49.8 compared to 73.3 for vehicle controls. Drug treatment was initiated at an age when the tumors were well-established. These results indicate that N-(2-((4-hydroxyphenyl)amino)pyrid-3-yl)-4-methoxybenzenesulfonamide shows significant *in vivo* activity in a spontaneous model of intestinal tumorigenesis.

N-(2-((4-hydroxyphenyl)amino)pyrid-3-yl)-4-methoxybenzenesulfonamide as a single agent demonstrated antitumor activity in multiple human tumor xenograft models *in vivo.* In several *in vivo* xenograft models (flank and orthotopic), once-a-day dosing of N-(2-((4-hydroxyphenyl)amino)pyrid-3-yl)-4-methoxybenzenesulfonamide demonstrated equal or greater efficacy compared to twice-a-day dosing. This superior QD efficacy was confirmed in a murine syngeneic model. Thus, administration of N-2-((4-hydroxyphenyl)amino)pyrid-3-yl)-4-methoxybenzenesulfonamide once a day appears to be sufficient to achieve maximal efficacy. In combination with 5-FU, cisplatin, docetaxel, and gemcitabine, N-(2-((4-hydroxyphenyl)amino)pyrid-3-yl)-4-methoxybenzenesulfonamide demonstrated an increase in antitumor activity in the HT-29 colon, Calu-6 NSCLC, MDA-MB-468 breast, and MiaPaCa2 pancreatic xenograft models respectively, compared to single agent alone.

NCI-H460 is a human non-small cell lung carcinoma derived cell line. It is MDR negative, has wild type p53, and contains an oncogenic K-ras mutation. N-(2-((4-hydroxyphenyl)amino)pyrid-3-yl)-4-methoxybenzenesulfonamide showed good efficacy in the NCI-H460 xenograft model. The mean tumor volume at day 13 was significantly different than the vehicle control (T/C = 58%). N-(2-((4-hydroxyphenyl)amino)pyrid-3-yl)-4-methoxybenzenesulfonamide also caused a delay in tumor growth with an ILS value of 32%. Paclitaxel and vincristine both lacked activity in this assay.

HCT-15 is a human colon carcinoma derived cell line. It is MDR positive, and expresses both mutant p53 and oncogenic K-ras. The HCT-15 cell line has one of the highest levels of mdr-1/P-glycoprotein expression of cells from the NCI tumor cell line panel. Paclitaxel and vincristine, which are both substrates for P-glycoprotein drug efflux pump, were not efficacious, while N-(2-((4-hydroxyphenyl)amino)pyrid-3-yl)-4-methoxybenzenesulfonamide was effective in inhibiting tumor growth.

### Effect of Cytotoxic Agents on the Growth of HCT-15 Human Colon Carcinoma Xenografts

| Compound | Dose^{a} (mg/kg/d) | Route Schedule | T/C^{b} (no. trials) | % ILS^{c} |
|---|---|---|---|---|
| ABT-751 | 50 | PO, QD, days 1-8 | 50 (2) | 34 |

N-(2-((4-hydroxyphenyl)amino)pyrid-3-yl)-4-methoxybenzenesulfonamide inhibited the growth of a variety of human tumor xenografts that were allowed to grow into established tumors prior to the initiation of treatment. As summarized below, activity was seen against established tumors derived from colon, breast and lung carcinomas. N-(2-((4-hydroxyphenyl)amino)pyrid-3-yl)-4-methoxybenzenesulfonamide was also active against a human pancreatic tumor xenograft grown in the orthotopic site.

### In vivo Activity of ABT-751 Against Staged and Orthotopic Human Tumor Xenografts

| Cell line | Oral Dose (mg/kg/d) | Schedule | % T/C | % ILS |
|---|---|---|---|---|
| Tumor Models | | | | |
| HT-29 colon carcinoma | 100 | QD, d 10-14, 20-24 | 28 | 75 |
| | 75 | QD, d 10-14, 20-24 | 43 | 50 |
| | 50 | QD, d 1-21 | 55 | 36 |
| Calu-6 lung carcinoma | 100 | QD, d 10-14, 20-24 | 37 | 65 |
| | 75 | QD., d 10-14, 20-24 | 50 | 58 |
| | 50 | QD, d 1-21 | 54 | 58 |
| MDA-MB-468 | 100 | QD, d 10-14, | 25 | 78 |
| breast carcinoma | | 20-24 | | |
| | 75 | QD, d 10-14, 20-24 | 33 | 52 |
| | 50 | QD, d 1-21 | 46 | 41 |

| Orthotopic Tumor Model | | | | |
|---|---|---|---|---|
| MiaPaCa-2 pancreatic | 100 | QD, d 1-5, 11-15 | 56 | n.d. |
| | 75 | QD, d 1-5, 11-15 | 79 | n.d. |
| HT-1376 bladder | 100 | QD, d 1-5, 11-15 | 63 | n.d. |
| | 100 | b.i.d., d 1-5, 11-15 | 86^{e} | n.d. |

N-(2-((4-hydroxyphenyl)amino)pyrid-3-yl)-4-methoxybenzenesulfonamide also showed antitumor activity against a variety of other human tumor xenografts in nude mice.

Antitumor activity was also seen in tumors derived from gastric, lung, breast, and oral carcinomas.

| Antitumor Activity of ABT-751 Against Human Tumor Xenografts | | | |
|---|---|---|---|
| Tumor Lines | Schedule | Dose (mg/kg/day) | T/C (%) |
| Gastric Cancer | | | |
| H-81 | Q5D × 5 | 300 | 40 |
| H-111 | Q1D × 19 | 80 | 36 |
| H-154 | Q5D × 5 | 300 | 71 |
| SC-2 | Q2D × 19 | 120 | 28 |
| SC-6 | Q5D × 5 | 500 | 22 |

| Colorectal Cancer | | | |
|---|---|---|---|
| H-143 | Q5D × 5 | 300 | 17 |
| COLO320DM | Q1D × 19 | 120 | 42 |
| WiDr | Q1D × 20 | 100 | 22 |

| Lung Cancer | | | |
|---|---|---|---|
| LC-376 | Q5D × 5 | 300 | 18 |
| LC-6 | Q5D × 5 | 450 | 31 |
| LC-11 | Q1D × 8 | 150 | 93 |
| LX-1 | Q1D × 20 | 120 | 37 |

| Breast Cancer | | | |
|---|---|---|---|
| H-31 | Q1D ×19 | 80 | 13 |
| MX-1 | Q5D × 5 | 450 | 21 |

| Oral Cancer | | | |
|---|---|---|---|
| KB | Q1D × 20 | 100 | 15 |

N-(2-((4-hydroxyphenyl)amino)pyrid-3-yl)-4-methoxybenzenesulfonamide was evaluated in 57 cancer subjects in single dose (16 subjects) and 5-day repeated dose regimens (41 subjects). The doses administered in the single dose segment were 80 to 480 mg/m²/day. In the 5-day repeated dose regimen (2-((4-hydroxyphenyl)amino)pyrid-3-yl)-4-methoxybenzenesulfonamide was given at 30 to 240 mg/m²/day for a single cycle. Pharmacokinetic data indicated that N-(2-((4-hydroxyphenyl)amino)pyrid-3-yl)-4-methoxybenzenesulfonamide plasma concentrations increased rapidly after dosing. The drug was eliminated with a half-life between 4 and 16 hours. The AUC increased proportionally with dose over the range of 30 to 480 mg/m²/day with no apparent accumulation. Adverse drug reactions from the single dose and 5-day repeated dose segments included nausea and vomiting, diarrhea, epigastric pain, ileus and evidence of peripheral neuropathy. For the single dose segment, the dose limiting toxicity (DLT) was Grade 3 peripheral neuropathy in 1 of 5 subjects at 480 mg/m²/day. In the 5-day repeated dose regimen the dose limiting toxicities were Grade 3 peripheral neuropathy in 1 of 4 subjects at 210 mg/m²/day and Grade 4 intestinal paralysis in 1 of 4 subjects at 210 mg/m²/day and in 1 of 6 subjects at 240 mg/m²/day.

In the 7-day QD regimen, the 250 mg QD dose has been determined to be the MTD, as dose limiting toxicities of peripheral neuropathy/ileus were reported in 2 of 6 subjects at the 300 mg QD dose. The MTD of the QD regimen given for 21 days was determined to be 200 mg as dose limiting toxicities of fatigue, anorexia and suspect small bowel obstruction were observed in 2/3 subjects in the 250 mg dose group.

A review of the safety data demonstrates no significant myelosuppression, renal or hepatic toxicity reported in any of the three studies.

In accordance with compositions, the tubulin β-subunit binders can be administered with or without an excipient. Excipients include encapsulating materials or additives such as absorption accelerators, antioxidants, binders, buffers, coating agents, coloring agents, diluents, disintegrants, emulsifiers, extenders, fillers, flavoring agents, humectants, lubricants, perfumes, preservatives, propellants, releasing agents, sterilizing agents, sweeteners, solubilizers, wetting agents and mixtures thereof. Excipients for solid dosage forms the tubulin β-subunit binders to be administered orally include agar, alginic acid, aluminum hydroxide, benzyl alcohol, benzyl benzoate, 1,3-butylene glycol, castor oil, cellulose, cellulose acetate, cocoa butter, corn starch, corn oil, cottonseed oil, ethanol, ethyl cellulose, ethyl laureate, ethyl oleate, gelatin, germ oil, glucose, glycerol, groundnut oil, isopropanol, isotonic saline, lactose, magnesium hydroxide, magnesium stearate, malt, olive oil, peanut oil, potassium phosphate salts, potato starch, propylene glycol, Ringer's solution, talc, tragacanth, water, safflower oil, sesame oil, sodium carboxymethyl cellulose, sodium laurel sulfate, sodium phosphate salts, soybean oil, sucrose, tetrahydrofurfuryl alcohol and mixtures thereof. Excipients for the tubulin β-subunit binders of this to be administered ophthalmically or orally in liquid dosage forms include 1,3-butylene glycol, castor oil, corn oil, cottonseed oil, ethanol, fatty acid esters of sorbitan, germ oil, groundnut oil, glycerol, isopropanol, olive oil, polyethylene glycols, propylene glycol, sesame oil, water and mixtures thereof. Excipients for the tubulin β-subunit binders to be administered osmotically include chlorofluorohydrocarbons, ethanol, water and mixtures thereof. Excipients for the tubulin β-subunit binders to be administered parenterally include 1,3-butanediol, castor oil, corn oil, cottonseed oil, germ oil, groundnut oil, liposomes, oleic acid, olive oil, peanut oil, Ringer's solution, safflower oil, sesame oil, soybean oil, U.S.P. or isotonic sodium chloride solution, water and mixtures thereof. Excipients for the tubulin β-subunit binders to be administered rectally or vaginally include cocoa butter, polyethylene glycol, wax and mixtures thereof.

A preferable excipient for the practice of this invention using N-(2-((4-hydroxyphenyl)amino)pyrid-3-yl)-4-methoxybenzenesulfomamide is shown hereinbelow.

### Formulation of N-(2-((4-hydroxyphenyl)amino)pyrid-3-yl)-4-methoxybenzenesulfonamide

| Ingredient | % w/w | purpose |
|---|---|---|
| ABT-751 | 30.0 | |
| microcrystalline cellulose NF (Avicel® PH101) | 15.8 | Filler |
| lactose monohydrate | 28.0 | Filler |
| povidone (USP, K29-32) | 8.0 | Binder |
| croscarmellose Na | 18.0 | Disintegrant |
| water | sufficient quantity | Binder Liquid |
| magnesium stearate | 0.2 | Lubricant |

A mixture of microcrystalline cellulose, N-(2-((4-hydroxyphenyl) amino)pyrid-3-yl) -4-methoxybenzenesulfonamide, lactose, and croscarmellose were granulated with a solution of povidone in water, dried, and milled. The milled product was blended with magnesium stearate.

The doses herein were made by filling capsules with the appropriate amount of blended product.

In accordance with routes of administration, the tubulin β-subunit binders may be administered orally, ophthalmically, osmotically, parenterally (subcutaneously, intramuscularly, intrasternally, intravenously), rectally, topically, transdermally, or vaginally. Orally administered solid dosage forms can be administered as capsules, dragees, granules, pills, powders, or tablets. Ophthalmically and orally administered dosage forms may be administered as elixirs, emulsions, microemulsions, suspensions, or syrups. Osmotically and topically administered dosage forms may be administered as creams, gels, inhalants, lotions, ointments, pastes, or powders. Parenterally administered dosage forms may be administered as aqueous or oleaginous suspensions. Rectally and vaginally dosage forms may be administered as creams, gels, lotions, ointments, or pastes.

For the practice of this disclosure, it is meant to be understood that while administration of drugs which bind to other than the colchicine binding site of tubulin β-subunits are preferentially administered parenterally, oral administration of drugs which bind to the colchicine binding site of tubulin β-subunits is more preferable than parenteral administration of the same drug.

The therapeutically acceptable amounts of the tubulin β-subunit binders and their dosing schedules depend on the recipient of treatment, the disease being treated and the severity thereof, the composition containing the tubulin β-subunit binder, the time of administration, the route of administration, the potency of the tubulin β-subunit binder, the rate of clearance of the tubulin β-subunit binder, and whether or not another drug is co-administered.

The daily amount of N-(2-((4-hydroxyphenyl)amino)pyrid-3-yl)-4-methoxybenzenesulfonamide to be administered orally in a continuous, once daily dose to adult patients having refractory solid tumors, is about 50 mg, about 100 mg, about 150 mg, about 200 mg, about 250 mg, or about 300 mg.

Preferably, about 250 mg of N-(2-((4-hydroxyphenyl) amino)pyrid-3-yl) -4-methoxybenzenesulfonamide is continuously administered orally once per day (QD) to adult patients having refractory solid tumors for the first 7 days of a 21 day schedule.

Preferably, about 200 mg of N-(2-((4-hydroxyphenyl)amino)pyrid-3-yl)-4-methoxybenzenesulfonamide is continuously administered orally once per day to adult patients having refractory solid tumors for the first 21 days of a 28 day schedule.

Preferably, about 200 mg of N-(2-((4-hydroxyphenyl)amino)pyrid-3-yl)-4-methoxybenzenesulfonamide is administered continuously orally once per day to adult patients having breast lung, kidney, or colon cancer, is for the first 21 days of a 28 day dosing schedule.

The daily amount of N-(2-((4-hydroxyphenyl)amino)pyrid-3-yl)-4-methoxybenzenesulfonamide to be administered orally in a continuous once per day dose to pediatric patients having refractory solid tumors, may be about 100 mg/mm², about 130 mg/mm², about 165 mg/mm², about 200 mg/mm², or about 250 mg/mm².

The daily amount of N-(2-((4-hydroxyphenyl)amino)pyrid-3-yl)-4-methoxybenzenesulfonamide to be administered orally in a continuous once per day dose to adult patients having refractory hematologic malignancies, may be about 100 mg/mm², about 125 mg/mm², and about 150 mg/mm².

The daily amount of N-(2-((4-hydroxyphenyl)amino)pyrid-3-yl)-4-methoxybenzenesulfonamide to be administered orally in continuous, twice daily (BID) doses to adult patients having refractory solid tumors, may be about 25 mg, about 50 mg, about 75 mg, about 100 mg, about 125 mg, about 150 mg, about 175 mg, about 200 mg, about 250 mg, or about 300 mg.

Preferably, about 175 mg of N-(2-((4-hydroxyphenyl)amimo)pyrid-3-yl)-4-methoxybenezesulfonamide is administered orally twice per day to adult patients having refractory solid tumors for the first 7 days of a 21 day schedule.

The daily amount of N-(2-((4-hydroxyphenyl)amino)pyrid-3-yl)-4-methoxybenzenesulfonamide to be administered orally in continuous twice per day doses to adult patients having refractory hematologic malignancies may be about 75 mg/mm², about 100 mg/mm², 125 mg/mm², 150 mg/mm², and 175 mg/mm².

The daily amount of N-(2-((4-hydroxyphenyl)amino)pyrid-3-yl)-4-methoxybenzenesulfonamide to be administered in continuous twice per day doses to pediatric patients having refractory solid tumor may be about 100 mg/mm² and about 130 mg/mm².

N-(2-((4-hydroxyphenyl)amino)pyrid-3-yl)-4-methoxybenzenesulfonamide may also be useful in the.treatment of disease when used alone or in combination with other therapies. For example, when used for the treatment of cancer, such compound may be administered alone or in combination with radiotherapy, hormonal agents, antibodies, antiangiogenics, COX-2 inhibitors, or other chemotherapeutic agents (cytotoxic or cytostatic) such as cisplatin, 5-fluorouracil, taxotere, docetaxel and gemcitabine.

Efficacy of N-(2-((4-hydroxyphenyl)amino)pyrid-3-yl)-4-methoxybenzenesulfonamide in combination with other chemotherapeutic drugs was tested in Calu-6 NSCL, MDA-MB-468 breast and HT-29 colon carcinoma xenografts which were grown in nude mice.

One objective was determination of the efficacy of N-(2-((4-hydroxyphenyl)amino)pyrid-3-yl)-4-methoxybenzenesulfonamide as a single agent and with cisplatin in the Calu-6 NSCL flank xenograft model.

Another objective was determination of the efficacy of N-(2-((4-hydroxyphenyl)amino)pyrid-3-yl)-4-methoxybenzenesulfonamide as a single agent and with docetaxel in the MDA-MB 468 breast flank xenograft model,

Still another objective was determination of the efficacy of N-(2-((4-hydroxyphenyl)amino)pyrid-3-yl)-4-methoxybenzenesulfonamide as a single agent and with 5-fluorouracil in the HT-29 colon flank xenograft model,

Still yet another objective was determination of the efficacy of N-(2-((4-hydroxyphenyl)amino)pyrid-3-yl)-4-methoxybenzenesulfonamide administered on 2 different schedules (once per day and twice per day) in the Calu-6 and HT-29 xenogaft model.

Following inoculation of tumor cells, tumors were passaged three times in mice before using the fragments to generate single cell suspensions. Multiple animals were used as donors. Single cell suspensions were generated by homogenizing the minced tumor tissue in RPMI 1640 media. Cell suspensions were washed three times to remove debris and other foreign materials. Viable cells were counted by trypan blue exclusion technique using a hemocytometer. Mice were injected subcutaneously with 0.5 mL of the cell suspension containing 1x10⁶ cells.

For continuous, 21 day, once per day dosing, 50 mg/kg/day was selected because administration at this dose was essentially without adverse side effects.

For determination of the efficacy of N-(2-((4-hydroxyphenyl)amino)pyrid-3-yl)-4-methoxybenzenesulfonamide in the Calu-6 Flank Xenograft model, tumor cells were inoculated subcutaneously into male nude mice on day 0. On day 10, mice bearing established tumors were size matched and grouped at about 233 mm³.

In the Calu-6 xenograft model, N-(2-((4-hydroxyphenyl)amino)pyrid-3-yl)-4-methoxybenzenesulfonamide administered as a single agent at 100 and 75 mg/kg/day on a (5 day on/5 day off)×2 cycle dosing schedule demonstrated significant antitumor activity. N-(2-((4-hydroxyphenyl)amino)pyrid-3-yl)-4-methoxybenzenesulfonamide at 50 mg/kg/day administered either on a once per day or twice per day schedule demonstrated similar efficacy. In combination with cisplatin, N-(2-((4-hydroxyphenyl)amino)pyrid-3-yl)-4-methoxybenzenesulfonamide demonstrated greater than additive responses with N-(2-((4-hydroxyphenyl)amino)pyrid-3-yl)-4-methoxybenzenesulfonamide.

For determination of the efficacy of N-(2-((4-hydroxyphenyl)amino)pyrid-3-yl)-4-methoxybenzenesulfonamide in the MDA-MB-468 Breast Flank Xenograft model, tumor cells derived from serially passaged tumor fragments were inoculated subcutaneously in female nude mice on day 0. On day 10, mice bearing established tumors were size matched and grouped at about 231 mm³.

As a single agent, N-(2-((4-hydroxyphenyl)amino)pyrid-3-yl)-4-methoxybenzenesulfonamide at 100 and 75 mg/kg/day for (5 days on/5 days off) ×2 dosing schedule, as well as 50 mg/kg/day administered daily, demonstrated dose-dependent antitumor activity in the MDA-MB 468 xenogaft model. In combination with docetaxel at 33.3 mg/kg/day, N-(2-((4-hydroxyphenyl)amino)pyrid-3-yl)-4-methoxybenzenesulfonamide demonstrated greater than additive responses with the doses of N-(2-((4-hydroxyphenyl)amino)pyrid-3-yl)-4-methoxybenzenesulfonamide tested.

For determination of the efficacy of N-(2-((4-hydroxyphenyl)amino)pyrid-3-yl)-4-methoxybenzenesulfonamide in the efficacy of N-(2-((4-hydroxyphenyl)amino)pyrid-3-yl)-4-methoxybenzenesulfonamide in the HT-29 Flank Xenograft model, tumor cells derived from serially passaged tumor fragments were inoculated subcutaneously in female nude mice on day 0. On day 10, mice bearing established tumors were size matched and grouped at about 236 mm³.

In the HT-29 colon xenograft model, N-(2-((4-hydroxyphenyl)amino)pyrid-3-yl)-4-methoxybenzenesulfonamide administered as a single agent at 100 and 75 mg/kg/day on a (5 days on/5 days off) ×2 dosing schedule demonstrated significant antitumor activity. N-(2-((4-hydroxyphenyl)amino)pyrid-3-yl)-4-methoxybenzenesulfonamide at 50 mg/kg/day administered either once per day or twice per day demonstrated similar efficacy in this model and confirmed the observation made with the Calu-6 model. In combination with 5-fluorouracil at 30 mg/kg/day, N-(2-((4-hydroxyphenyl)amino)pyrid-3-yl)-4-methoxybenzenesulfonamide at the higher doses demonstrated additive responses.

To summarize, the efficacy of N-(2-((4-hydroxyphenyl)amino)pyrid-3-yl)-4-methoxybenzenesulfonamide was tested in the Calu-6 NSCL, MDA-MB-468 breast and HT-29 colon xenograft models. As a single agent, N-(2-((4-hydroxyphenyl)amino)pyrid-3-yl)-4-methoxybenzenesulfonamide demonstrated dose-dependent efficacy in all three xenograft models. Efficacy of N-(2-((4-hydroxyphenyl)amino)pyrid-3-yl)-4-methoxybenzenesulfonamide administered once per day and twice per day was tested in the Calu-6 NSCL and HT-29 colon xenograft models and demonstrated equal efficacy when administered in either once per day or twice per day for 21 days in both models.

Accordingly, N-(2-((4-hydroxyphenyl)amino)pyrid-3-yl)-4-methoxybenzenesulfonamide in combination with cisplatin (Calu-6 NSCLC), docetaxel (MDA-MB-468) or 5-FU (HT-29) showed equal to or greater than additive efficacy compared to single agents alone.

To evaluate the pharmacokinetics of representative extended dosing schedules of N-(2-((4-hydroxyphenyl)amino)pyrid-3-yl)-4-methoxybenzenesulfonamide, 43 patients were enrolled. The tumor types studied were colorectal (23), sarcoma (5), mesothelioma (3), salivary gland (2), endometrial (2), unknown (2), hepatoma (1), melanoma (1), renal cell (1), lung (1), ovary (1), and granulosa cell (1). Patients were treated once or twice per day for 21 days with N-(2-((4-hydroxyphenyl)amino)pyrid-3-yl)-4-methoxybenzenesulfonamide followed by a 7-day period where no drug was received. Doses were escalated by 50 mg/day (25 mg twice per day). Three patients were initially treated at each dose level. If dose-limiting toxicity was observed in cycle one, three more patients were added to that dosing schedule. If additional patients experienced dose-limiting toxicity, on occasion the dose level was expanded to nine patients to further assess tolerability. Response assessment was performed every two cycles.

For 134 patients tested, at doses of about 200 mg QD, 250 mg QD, 300 mg QD, 125 mg BID, 150 mg BID, and 175 mg BID, 16 reported anemia, of which 11 were Grades I or II and 5 were Grades (III or IV); 1 reported Grade I or II alpoecia; 8 reported Grade I or II neutropenia; and none reported fluid retention, for which Grade I is defined as mild, Grade II is defined as moderate, Grade III is defined as severe, Grade IV is defined as life threatening.

The foregoing is merely illustrative of the invention and is not intended to limit the invention to the disclosed embodiments. Variations and changes obvious to one skilled in the art are intended to be within the scope and nature of the invention which are defined in the appended claims.

## Claims

1. A pharmaceutical composition comprising N-(2-((4-hydroxyphenyl)amino)pyrid-3-yl)-4-methoxybenzenesulfonamide and at least one cancer drug selected from the group consisting of cisplatin, docetaxel, and 5-fluorouracil.

2. A pharmaceutical composition according to claim 1 for use in the treatment of cancer.

3. Use of N-(2-((4-hydroxyphenyl)amino)pyrid-3-yl)-4-methoxybenzenesulfonamide and at least one additional drug selected from the group consisting of cisplatin, docetaxel, and 5-fluorouracil for the manufacture of a medicament for treating cancer in a human wherein said medicament is to be administered in a therapeutically acceptable amount.

## Patentansprüche

1. Eine pharmazeutische Zusammensetzung umfassend N-(2-((4-Hydroxyphenyl)amino)pyrid-3-yl)-4-methoxybenzensulfonamid und mindestens ein Krebs-Medikament gewählt aus der Gruppe bestehend aus Cisplatin, Docetaxel und 5-Fluoruracil.

2. Eine pharmazeutische Zusammensetzung gemäß Anspruch 1 für die Verwendung in der Behandlung von Krebs.

3. Verwendung von N-(2-((4-Hydroxyphenyl)amino)pyrid-3-yl)-4-methoxybenzensulfonamid und mindestens einem zusätzlichen Arzneimittel gewählt aus der Gruppe bestehend aus Cisplatin, Docetaxel und 5-Fluoruracil für die Herstellung eines Medikaments für die Behandlung von Krebs in einem Menschen, worin das Medikament in einer therapeutisch verträglichen Menge zu verabreichen ist.

## Revendications

1. Composition pharmaceutique comprenant du N-(2-((4-hydroxyphényl)amino)pyrid-3-yl)-4-méthoxybenzènesulfonamide et au moins un médicament anticancéreux choisi dans le groupe consistant en cisplatine, docétaxel et 5-fluorouracile.

2. Composition pharmaceutique selon la revendication 1 destinée à être utilisée dans le traitement du cancer.

3. Utilisation du N-(2-((4-hydroxyphényl)amino)pyrid-3-yl)-4-méthoxybenzènesulfonamide et d'au moins un médicament supplémentaire choisi dans le groupe consistant en cisplatine, docétaxel et 5-fluorouracile pour la fabrication d'un médicament pour le traitement du cancer chez un humain où ledit médicament est destiné à être administré en une quantité thérapeutiquement acceptable.
